(19) **Europäisches Patentamt / European Patent Office / Office européen des brevets**

(11) **EP 1 863 831 B1**

(12) **EUROPÄISCHE PATENTSCHRIFT**

(45) Veröffentlichungstag und Bekanntmachung des Hinweises auf die Patenterteilung:
**18.07.2012 Patentblatt 2012/29**

(21) Anmeldenummer: **06723222.3**

(22) Anmeldetag: **06.03.2006**

(51) Int Cl.:
*B01D 57/02* (2006.01)  *C07K 1/26* (2006.01)
*B01D 61/42* (2006.01)  *C07K 1/24* (2006.01)
*C07K 1/34* (2006.01)

(86) Internationale Anmeldenummer:
**PCT/EP2006/002012**

(87) Internationale Veröffentlichungsnummer:
**WO 2006/097212 (21.09.2006 Gazette 2006/38)**

(54) **ELEKTROFILTRATIONSVERFAHREN**

ELECTROFILTRATION METHOD

PROCEDE D'ELECTROFILTRATION

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **18.03.2005 DE 102005012594**

(43) Veröffentlichungstag der Anmeldung:
**12.12.2007 Patentblatt 2007/50**

(73) Patentinhaber: **Bayer Technology Services GmbH 51368 Leverkusen (DE)**

(72) Erfinder:
• **DUDZIAK, Gregor Oakland, CA 94602 (US)**

• **TRAVING, Michael 51399 Burscheid (DE)**
• **MUTTER, Martina 51063 Köln (DE)**

(56) Entgegenhaltungen:
**EP-A- 0 091 784    EP-A- 0 369 945**
**DE-A1- 10 253 483**

• **HUOTARI H M ET AL: "Electrically enhanced crossflow membrane filtration of oily waste water using the membrane as a cathode" JOURNAL OF MEMBRANE SCIENCE, ELSEVIER SCIENCE, AMSTERDAM, NL, Bd. 156, Nr. 1, 24. April 1999 (1999-04-24), Seiten 49-60, XP004163131 ISSN: 0376-7388**

**Beschreibung**

Die Erfindung betrifft ein Verfahren zur Verbesserung der Selektivität und Produktivität bei der Aufreinigung von geladenen Makromolekülen und Partikeln durch Elektrofiltration mittels Membranen im elektrischen Feld. Dabei wird das Verhältnis aus oben genanntem elektrischen Feld und dem Permeatfluss durch die Membran konstant gehalten und das elektrische Feld auf der Feedseite der Membran (Feedkanal) in Abhängigkeit von der gemessenen Leitfähigkeit der Feedlösung geregelt.

Der Einsatz der Membran-Elektrophorese sowie der Elektrofiltration als präparative Trenntechniken wird seit den 50her Jahren untersucht.

Bei beiden Verfahren werden Separationskammern durch semipermeable Membranen voneinander getrennt.

Bei der Membranelektrophorese findet eine Migration gelöster Ionen und dispergierter, geladener Partikel und Agglomerate im elektrischen Feld statt, aber im Wesentlichen kein Flüssigkeitsstrom durch die Separationsmembran. Triebkraft für den Stofftransport ist das elektrische Feld.

Bei der Elektrofiltration wird neben dem elektrischen Feld eine hydrostatische Druckdifferenz zwischen den Separationskammern aufgebaut, so dass ein Flüssigkeitsstrom durch die Separationsmembran induziert wird. Triebkräfte für den Stofftransport sind somit das elektrische Feld als auch die Druckdifferenz zwischen den Separationskammern.

Die Elektrofiltration wurde erstmals von Bier, 1959, US A 3 079 318, als so genannte "Forced Flow Electrophoresis" erwähnt. Sie wurde angewendet, um die Verblockung von Membranen bei der Filtration von Hefe, aber auch bei der Abreicherung von Zellen und Albumin aus Blut zu verringern.

Durch Huotari H.M. et al. wird in "Electrically enhanced crossflow membrane filtration of oily waste water using the membrane as a cathode" (Journal of Membrane Science, Elseviers Science Amsterdam, NL, 156 (1), 24.04.1999, Seiten 49-60) der Einfluss des elektrischen Feldes auf elektrophoretische Membranfiltrationsverfahren zur Trennung ölhaltiger Gemische allgemein beschrieben.

US A 3 989 613 (Gritzner 1976) beschreibt ein Membran-Elektrophorese-Verfahren, bei der beide Ströme (Feed- und Permeat) parallel strömen. Die Strömungsgeschwindigkeit in den Separationskammern ist sehr niedrig (<0,01cm/s). Daraus ergibt sich ein verhältnismäßig schlechter Wärmeaustrag und ein nicht-turbulenter Fluss, der wiederum zu ungewünschter Konzentrationspolarisation führt. Eingesetzt werden nicht-selektive Separationsmembranen.

Eine weitere Veröffentlichung zur Elektrofiltration ist die Dissertation von Tison, 1986, Selective cascade electrofiltration. Diese befasst sich mit der Abtrennung von IgG aus humanem Blutplasma mittels Elektrofiltration. Da das IgG- abgereicherte Plasma jedoch wieder in den Menschen zurückgeführt werden soll, wurde eine Betriebsweise bei physiologischen Salzkonzentration gewählt. Eine Entsalzung wird also weder vor noch während des Prozesses durchgeführt, die Betriebsweise ist stromstärkekonstant und druckkonstant. Aufgrund der hohen Leitfähigkeit des Blutplasmas können jedoch nur niedrige Produktivitäten realisiert werden.

Die Methode der Elektrofiltration wurde von Perry et al., 1992, US A 5 087 338 weiter untersucht. Perry beschreibt die Trennleistung der Elektrofiltration mittels proteinhaltiger Modelllösungen hinsichtlich der Selektivität. Dabei können Biomoleküle, deren isoelektrische Punkte eine Differenz von 0,1 haben, getrennt werden. In den offenbarten Beispielen wird das Verfahren stromstärkekonstant betrieben. Da die Leitfähigkeit der stark verdünnten Feedlösungen sehr niedrig ist und sich kaum von der der übrigen Puffer im Permeatkanal und in den Elektrodenräumen unterscheidet, können kaum Leitfähigkeitsänderungen in der Feedlösung auftreten. Hierbei handelt es sich um nicht praxisrelevante Anwendungen im Labormaßstab mit Modellsubstanzen. In der Praxis hingegen treten Änderungen der Leitfähigkeit ein.

Die EP 0 091 784 A2 beschreibt die Verwendung von elektrophoretischen Verfahren, wie sie in der US A 5 087 338 beschrieben sind, zur Abtrennung mono- und polyklonaler Antikörper.

Laustsen erläutert in US A 5 437 774 von 1993 die so genannte "Elektrodialyse von Molekülen mit großem Molekulargewicht". Dabei beschreibt er ausgehend von der Trenntechnik der Dialyse den möglichen Aufbau der Zelle mit einer oder mehreren Separationsmembranen und verschiedenen Kammern für Diluat und Konzentrat. Die Membranen können dabei ebenso ungeladene, wie auch Ionentauschermembranen sein. Der Prozess kann ohne Permeatfluss durch die Membran (klassische Membranelektrophorese) oder mit einem durch Druck erzwungenen Fluss, im weiteren auch beschrieben als Elektrofiltration, betrieben werden. Diese kann in oben genanntem Aufbau oder auch mit mehreren Kanälen parallel betrieben werden, wodurch die Membranfläche und also die Wirtschaftlichkeit des Verfahrens erhöht wird, ebenso wie durch eine mögliche Durchführung mehrerer Trennschritte hintereinander. In einem Ausführungsbeispiel beschreibt Laustsen die Trennung von BSA und Hämoglobin. Die Fahrweise ist spannungskonstant und druckkonstant (Transmembrandruck von 67mbar) bei vergleichsweise hoher Spannung (160V) und geringer Überströmung (0,1 bis 0,2m/s). Diese Parameterkombination würde im technischen Maßstab zu Problemen mit der Wärmeabfuhr und zu Konzentrationspolarisation der Proteine an den Separationsmembranen führen.

Eine Patentanmeldung der Firma Gradipore (1999, WO A 99/62937) namens "Purification of Antibodies" beschreibt die Aufreinigung von Antikörpern, speziell monoklonalen Antikörpern, aus Bauchfellwasser von Mäusen mithilfe der Membran-Elektrophorese und der sog. Gradiflow Technologie (AU A 601040). Dabei wird der pH-Wert der Lösung so eingestellt, dass der Antikörper ungeladen vorliegt und die geladenen Verunreinigungen elektrophoretisch durch die Membran abgereichert werden.

Um die Aufreinigung des Antikörpers, also die Abreicherung der Verunreinigungen möglichst vollständig durchzuführen, kann der Versuch unterbrochen und das Konzentratvolumen ausgetauscht werden oder der Versuch zweistufig durchgeführt werden, wobei der pH-Wert der Lösung, die Porengröße der Membran und die Richtung des elektrischen Feldes entsprechend angepasst werden. Das Verfahren wird spannungskonstant bei 200V durchgeführt. Die daraus resultierende hohe elektrische Feldstärke würde im technischen Maßstab zu Problemen mit der Wärmeabfuhr und zu Konzentrationspolarisation und Proteindenaturierung an der Membran führten.

Ein ähnliches Verfahren wird 2004 in DE A 102 534 83 offenbart. In dieser Anmeldung wird erstmals eine Laboranlage im skalierbaren Maßstab vorgestellt, (Vorlagevolumina von 1L). Durch eine Strömungsgeschwindigkeit von 5,2cm/s in den Separationskammern kann ein genügend hoher Wärmeaustrag gewährleistet werden. Membranen bilden durch Diffusion von Ionen aus der Membran in ein wässriges Medium elektrisch geladene Doppelschichten an der fest-flüssig-Grenzfläche aus. Bei der Verwendung von poröser Membranen wird daher im elektrischen Feld ein elektroosmotischer Druck induziert, der einen Flüssigkeitsstrom durch ebendiese Membran bewirkt. Um diesen elektroosmotischen Fluss zu kompensieren, wird in diesem Verfahren einer der Separationsräume mit einem genau abgestimmten elektroosmotischen Druck überlagert. Durch die Kompensation des elektroosmotischen Flusses kann die Produktivität und vor allem die Selektivität eines elektrophoretischen Verfahrens signifikant verbessert werden.

Weiterhin beschreibt DE A 0 40 07 848, "Vorrichtung und Verfahren für die Membranelektrophorese und Elektrofiltration", ein Membranmodul, mit welchem eine Proteintrennung mittels Membran-Elektrophorese oder Elektrofiltration bevorzugt durchgeführt werden kann. Vorteil hierbei ist die geschlossene, fest gefügte Ausführung, die hohe Überströmungsgeschwindigkeiten in den Separationskanälen, eine Minimierung der Toträume und eine hohe Dichtigkeit gewährleisten Die Offenlegung beschreibt zwar auch den grundsätzlichen Einsatz der Module zur Elektrofiltration. Wahl und Optimierung der Betriebsparameter, wie etwa hydrostatischer Druck und elektrische Feldstärke, sind nicht Thema dieser Offenlegung.

Üblicherweise werden Antikörper nach einem bestimmten, im Patent US A 5 429 746, Shadle et al. "Antibody Purification" von 1995, näher erläuterten Verfahren durchgeführt.

Dabei gibt es eine bestimmte Anzahl von Aufreinigungsschritten, die in der einen oder anderen Kombination und Reihenfolge verwendet werden.

Kernschritt dieses Verfahrens ist die Produktgewinnung und Aufkonzentrierung aus dem Fermentationsmedium. Dieser besteht in der Regel aus einer Aufkonzentrierung und Umpufferung mittels Ultrafiltration, gefolgt von einem Affinitätschromatographie-Schritt. Letzterer ist zwar hochselektiv, weist aber im technischen Maßstab eine Reihe von Nachteilen auf. Zu nennen sind etwa:

a) der hohe Preis des Chromatographiemediums

b) die Gefahr der Auswaschung des toxischen Liganden Protein A, die eine Nachbehandlung erfordert (in der Regel einen zusätzlichen Chromatographieschritt)

c) der niedrige pH-Wert bei der Elution (<3), der einen weiteren Verfahrensschritt zur Umpufferung nach sich zieht, um das Medium folgenden Verfahrensschritten unterziehen zu können.

Der Productgewinnung (Capturing) folgen in der Regel mindestens zwei weitere Chromatographieschritte (Purification und Polishing).

Die bisher beschriebenen Verfahren zur Aufreinigung von Antikörpern weisen somit eine Reihe von Nachteilen auf:

Klassische Verfahren:

Die herkömmliche Aufreinigung von Proteinen aus einer Fermentation erfolgt mindestens mit der oben beschriebenen Anzahl an Prozessschritten. Diese komplexen Prozesse sind mit hohen Kosten verbunden, die sich durch das erfindungsgemäße neü Verfahren reduzieren lassen.

Elektrophoretische Verfahren allgemein:

Membran-Elektrophorese und Elektrofiltration wurden bisher nicht im technischen oder Produktionsmassstab durchgeführt. Zudem wurden in der Mehrzahl der Fälle Modelllösungen mit einer Leitfähigkeit gewählt, die deutlich unter der einer Flüssigkeit mit physiologischen Salzkonzentrationen liegt, wie etwa der eines Zellkulturmediums.

Elektrofiltration:

Versuche zur Elektrofiltration wurden in der Vergangenheit wahlweise stromstärke- oder spannungskonstant betrieben. Da biotechnologische Medien und Produktlösungen im Prozess eine verhältnismäßig hohe Leitfähigkeit besitzen, ist eine Absenkung derselben während der Elektrofiltration erforderlich, um eine hohe Mobilität im elektrischen Feld und somit eine hohe Produktivität des Prozesses zu erzielen. Um also einen solchen Prozess jenseits von Labormaßstab und Modelllösung überhaupt realisieren zu können, ist folgendes zu beachten:

Neben der Produktivität ist die Selektivität eine weitere Zielgröße des Elektrofiltrations-Prozesses. Die Selektivität der Separation hängt wiederum maßgeblich vom druckgetriebenen Stofftransport durch die Separationsmembran sowie von der Migrationsgeschwindigkeit der Proteine im Feedkanal ab. Die Migration gelöster Ionen im elektrischen Feld kann dem druckgetriebenen Stofftransport entweder gleichgerichtet oder entgegengerichtet sein. Die Zielgrößen Produktivität und Selektivität können für den gegebenen Prozess nun mittels der Betriebsgrößen Spannung und hydrostatischer Druck optimiert werden. Die Betriebsgrößen werden dabei abhängig von den physikalischen Eigenschaften der gelösten Komponenten (zum Beispiel isoelektrischer Punkt, Molmasse, effektive Ladung bei gewähltem pH) und des Mediums (zum Beispiel Leitfähigkeit) gewählt. Ändert sich nun aber die Leitfähigkeit des Mediums im Feedkanal, so kann der optimale Betriebspunkt bei konstanten Betriebsparametern nicht aufrechterhalten werden.

Die Problematik soll am Beispiel eines Elektrofiltration-Verfahrens erläutert werden, bei dem ein monoklonaler Antikörper in der Feedlösung zurückgehalten werden soll, während die Nebenkomponenten durch die Separationsmembran abgereinigt werden. Der pH-Wert der Lösung wird dabei so gewählt, dass der monoklonale Antikörper eine negative Ladung aufweist. Hydrostatischer Druck und elektrische Spannung werden nun dergestalt gewählt, dass die Migrationsgeschwindigkeit des monoklonalen Antikörpers im elektrischen Feld mindestens der Permeatgeschwindigkeit des Mediums durch die Membran entspricht und dieser entgegengerichtet ist. Nebenkomponenten, die neutral oder positiv geladen sind, werden durch die Separationsmembran abgereichert.

Wird nun eine Lösung für den Permeatraum mit physiologischer Leitfähigkeit eingesetzt, der der des Zellkulturmediums entspricht (ca. 10 mS/cm), so kann das optimale Verhältnis von Migrationsgeschwindigkeit des Antikörpers und Permeationsgeschwindigkeit des Mediums durch die Separationsmembran über den gesamten Versuchsverlauf aufrechterhalten werden. Die Selektivität liegt somit während des gesamten Versuchsverlaufs konstant am Optimum Aufgrund der hohen Leitfähigkeiten und eines limitierten Wärmeaustrags lässt sich jedoch nur eine verhältnismäßig niedrige Produktivität erzielen.

Wird allerdings eine Lösung niedriger Leitfähigkeit für den Permeatraum eingesetzt, um das Zellkulturmedium im Feedkanal zu entsalzen, so passt sich die Leitfähigkeit des Mediums im Feedkanal während des Versuchsverlaufs der des Permeatkanals an. Bei spannungskonstantem Betrieb steigt dabei die Migrationsgeschwindigkeit des Antikörpers aufgrund der steigenden elektrischen Feldstärke im Feedkanal. Eine steigende Produktivität kann bei konstantem hydrostatischem Druck allerdings kaum realisiert werden. Wird dagegen der hydrostatische Druck von Versuchsbeginn an höher gewählt, so wird zwar eine erhöhte Produktivität erzielt. Die Selektivität des Verfahrens liegt allerdings zu Beginn des Verfahrens niedriger, und die Antikörper-Ausbeute ist verringert.

Der Erfindung liegt also die Aufgabe zugrunde ein Verfahren zur Aufreinigung von geladenen Makromolekülen aus Feedlösungen mit sich im Verfahren verändernden Leitfähigkeiten bereitzustellen, dessen Betriebsparameter so angepasst werden können, dass die Zielgrößen Produktivität und Selektivität über den gesamten Versuchsverlauf an den gewählten Optima liegen.

Dabei soll das makromolekülhaltige, insbesondere proteinhaltige, Medium, insbesondere ein antikörperhaltiges Fermentationsmedium, mittels Elektrofiltration aufkonzentriert und die im Fermentationsüberstand befindlichen Nebenkomponenten vom Zielprotein abgetrennt werden.

Gegenstand der Erfindung ist also ein Verfahren zur Aufreinigung von geladenen Makromolekülen und Partikeln aus Feedlösungen mit sich im Verfahren verändernden Leitfähigkeiten durch Elektrofiltration mittels Membranen im elektrischen Feld, mit dem die Selektivität und Produktivität während des gesamten Verlaufs verbessert wird, indem das Verhältnis aus oben genanntem elektrischen Feld und dem Permeatfluss durch die Membran konstant gehalten wird und das elektrische Feld auf der Feedseite der Membran (Feedkanal) in Abhängigkeit von der gemessenen Leitfähigkeit der Feedlösung geregelt wird.

Der Permeatfluss durch die Separationsmembran wird dergestalt geregelt, dass ein konstantes, optimales Verhältnis von Feldstärke zu Permeatfluss erzielt wird.

Soll ein Produkt aus einer Lösung mit physiologischer Salzkonzentration, also relativ hoher Leitfähigkeit, aufgereinigt werden, so werden bevorzugt im Zuge der Absenkung der Leitfähigkeit im Feedkanal durch Entsalzung sowohl die Feldstärke im Feedkanal als auch der Permeatfluss gleichzeitig so angepasst, insbesondere erhöht, dass beide Parameter immer in einem konstanten Verhältnis züinander stehen.

Dabei wird das elektrische Feld E allgemein beschrieben als

$$E = \frac{U}{d}$$

wobei U die anliegende Spannung und d der Elektrodenabstand ist.

Um die Feldstärke im Feedkanal zu berechnen, wird die folgende Gleichung eingesetzt:

$$U_{Feed} = R_{Feed} \cdot I = \frac{1}{LF_{Feed}} \cdot I$$

mit der Leitfähigkeit LF und der Stromstärke I.

Für das elektrische Feld im Feedkanal folgt:

$$E_{Feed} = (\frac{1}{LF_{Feed}} \cdot I) / d$$

Die Leitfähigkeit des Feed-Mediums wird in einer Durchflussmesszelle gemessen. Die anzulegende Stromstärke kann berechnet und entsprechend eingestellt werden. Diese Regelung wird mit Hilfe einer Automatisierungseinheit und eines extern ansteürbaren Netzteils realisiert.

Der Permeatfluss wird in einem konstanten Verhältnis zur Feldstärke im Feedkanal gehalten. Die Regelung des Permeatflusses kann zum Beispiel mittels Drucküberlagerung des Vorlagegefäßes oder durch entsprechende Einstellung der Volumenströme in Feed- und Permeatkanal erfolgen.

Die oben beschriebene Abstimmung von elektrischer Feldstärke im Feedkanal und dem Permeatfluss über die Membran wurde an einer Laboranlage realisiert.

Durch den Betrieb der Elektrofiltration bei einem konstanten Verhältnis der Feldstärke im Feedkanal zum Permeatfluss, sowie durch die Optimierung der Betriebsparameter konnten Produktivität und Selektivität bei der Aufreinigung von Makromolekülen überraschend verbessert werden.

Das erfindungsgemäße Verfahren zur Aufreinigung von geladenen Makromolekülen und Partikeln durch Elektrofiltration mittels Membranen im elektrischen Feld, wird so betrieben, dass das Verhältnis aus oben genanntem elektrischen Feld im Feedkanal und dem Permeatfluss durch die Membran, z.B. durch eine automatisierte Kontrolle, konstant gehalten wird.

Das elektrische Feld beträgt dabei 500 bis 5000 V/m, bevorzugt 1000 bis 2000 V/m. Die Feldstärke ist dabei so einzustellen, dass das erzeugte Feld ausreicht, um die abzureichernden geladenen Moleküle in angemessener Zeit aus dem Feed- in den Permeatkanal zu bewegen und dabei gleichzeitig die entgegengesetzt geladenen und dadurch zurückzuhaltenden Moleküle im Feedkanal zu halten. Die geeignete Feldstärke muss in ersten Vorversuchen zur Elektrofiltration ermittelt werden. Geeignet bedeutet in diesem Fall neben den oben beschriebenen Bedingungen auch, die Temperatur zwischen 0 und 40°C, insbesondere zwischen 6 und 25°C zu halten, um die geladenen Makromoleküle, insbesondere Biomoleküle, nicht zu denaturieren.

Der Permeatstrom liegt dabei zwischen 5 und 100, bevorzugt zwischen 10 und 30 L/(h*m$^2$).

Mit diesem Verfahren können eine oder mehrere der folgenden makromolekularen, insbesondere biomakromolekularen Substanzen behandelt werden: Proteine, Peptide, DNA, RNA, Oligonucleotide, Oligo- und Polysaccharide, Viren, Virenbestandteile, Zellen, Zellenbestandteile, Enantiomere, Diastereomere.

Dabei kann es sich bei den oben genannten biomakromolekularen Substanzen um Proteine, insbesondere um monoklonale oder polyklonale Antikörper handeln.

Die eingesetzte Membran weist eine Porengröße von 1 bis 1000 nm, vorzugsweise 10 bis 500 nm, auf und besteht bevorzugt aus einem der folgenden Werkstoffe: Celluloseester, Polyacrylnitril, Polyamid, Polyether, Polyethersulfon, Polypropylen, Polysulfon, Polyvinylalkohol, Polyvinylidenfluorid, oder aus Aluminiumoxid, Siliciumoxid, Titanoxid, Zirkonoxid sowie Mischkeramiken aus den oben genannten Oxiden

Die Leitfähigkeit der Ausgangslösung, die eine Mischung verschiedener geladener Makromoleküle oder Partikel, bevorzugt jedoch der Zellkulturüberstand einer Fermentation ist, liegt gewöhnlich zwischen 20 mS/cm und 0,5 mS/cm, besonders zwischen 12 und 1,5 mS/cm.

Im Verlauf des Verfahrens wird die Leitfähigkeit bevorzugt auf 3 bis 0,5 mS/cm abgesenkt.

Der Prozess kann auch als kontinuierlich betriebenes Verfahren durchgeführt werden. Kontinuierlich bedeutet dabei zum einen die Realisierung einer Verschaltung mehrerer Elektrophorese- Module in Kaskaden- oder Tannenbaumstruktur, wobei sich die oben näher beschriebenen Betriebsparameter während des Anfahrvorgangs zeitlich und danach räumlich, also in den verschiedenen Membranmodulen, verändern. Zum anderen bedeutet kontinuierliche Fahrweise der Betrieb eines Elektrophorese-Moduls bei kontinuierlicher Ab- und Zufuhr eines Teilstroms, entsprechend der Diafiltration. Dabei ändern sich die Betriebsparameter ebenfalls zeitlich während des Anfahrvorgangs bis ein stationärer Zustand erreicht ist.

Der Zellkulturüberstand kann direkt, bevorzugt jedoch durch Vorfiltration mittels einer Mikrofiltrationsmembran von Zellrückständen befreit, eingesetzt werden.

Der Zellkulturüberstand kann mittels Ultrafiltration aufkonzentriert sein.

Die verwendeten Elektrolyte im Feed-, Permeat- und Elektrodenspülkreislauf bestehen aus einer Kombination aus schwachen Säuren und schwachen Basen, schwachen Säuren und starken Basen oder starken Säuren und schwachen Basen.

Dabei enthalten die Elektrolytlösungen vorzugsweise eine oder mehrere der folgenden Substanzen: Borsäure, Phosphorsäure, N-2-(Acetamido)-2-aminoethansulfonsäure, N-2-(Acetamido)imino-diessigsäure, Alanin, 2-Amino-2-methyl-1,3-propandiol, Ammoniak, N,N-Bis(2-hydroxyethyl)-2-aminoethansulfonsäure, N,N-Bis(2-hydroxyethyl)glycin, 2,2-Bis(hydroxyethyl)-iminotris(hydroxymethyl)methan, 2-(Cyclohexylamino)ethansulfonsäure, Essigsäure, Glycin, Glycylglycin, 2-[4-(2-Hydroxyethyl)1-piperazinyl]ethansulfonsäure, 3-[4-(2-Hydroxyethyl)1-piperazinyl]propansulfonsäure, Histidin, Imidazol, Milchsäure, 2-Morpholinoethansulfonsäure, 2-Morpholinopropansulfonsäure, Piperazin-1,4-bis(2-ethansulfonsäure), N-[Tris(hydroxymethyl)-methyl]-2-aminoethansulfonsäure, N-(Tris(hydroxymethyl)-methyl]glycin, Triethanolamin, Tris(hydroxymethyl)aminomethan, Zitronensäure.

Der pH-Wert der Puffersysteme liegt zwischen 3 und 11.

Bei der Elektrolytlösung handelt es sich um eine wässrige Lösung zur Bereitstellung einer gewissen Ionenfracht, die den Ladungstransport durch das elektrische Feld ermöglicht, sowie um ein Puffersystem zur definierten Einstellung und Konstanthaltung des pH-Wertes in der Lösung.

In Vorversuchen ist die Stabilität der Makromoleküle zu ermitteln, indem diese in den verschiedenen Elektrolytlösungen bei verschiedenen Konzentrationen, Salzgehalten und Temperaturen angesetzt werden und das Ausfallen oder Agglomerieren der Makromoleküle, bevorzugt der Biomoleküle beobachtet wird. Die Beobachtung verläuft dabei optisch und per Messung des Proteingehaltes über ein chromatographisches Analysenverfahren.

Dem Verfahren zur Elektrofiltration kann, wenn nötig, ein weiterer Membranelektphoreseschritt folgen, der zur weiteren Aufreinigung eines Makromoleküls unter anderen Verfahrensbedingungen dient, zum Beispiel bei verändertem pH-Wert.

Das Verfahren kann ebenfalls betrieben werden als eine Kombination aus den Schritten Entsalzung und Aufkonzentrierung ohne Anlegen eines elektrischen Feldes, gefolgt von einer Elektrofiltration. Beide Schritte können in derselben Anlage mit denselben Membranmodulen durchgeführt werden.

Das Verfahren kann ebenfalls betrieben werden als eine Kombination einer Membranelektrophorese zur teilweisen Entsalzung oder zur Umpufferung und anschließender Elektrofiltration zur Trennung der geladenen Makromoleküle.

Alle beschriebenen Verfahrensvarianten können sowohl in Batch-, also diskontinuierlich, als auch in Feed-& Bleed Fahrweise, also kontinuierlich, betrieben werden.

Das Verfahren in allen beschriebenen Varianten kann zur Prozessintensivierung bei der Aufreinigung von Biomolekülen, insbesondere Proteinen, ganz besonders Antikörpern, eingesetzt werden.

Die Erfindung wird nachfolgend anhand der Figuren und der Beispiele, welche jedoch keine Beschränkung der Erfindung darstellen, näher erläutert bzw. illustriert.

Es zeigen:

Fig.1    das Schema einer Elektrofiltrations-Anlage mitautomatischer Regelung des elektrischen Feldes sowie der Einrichtung zur Druckkontrolle, die der Regelung des Permeatflusses dient.

Fig.2    das Schema eines Membranmoduls ausgeführt in Mehrkanalbauweise

Fig. 3    SDS-PAGE Gel unter reduzierenden Bedingungen zum Beispiel

## Beispiele

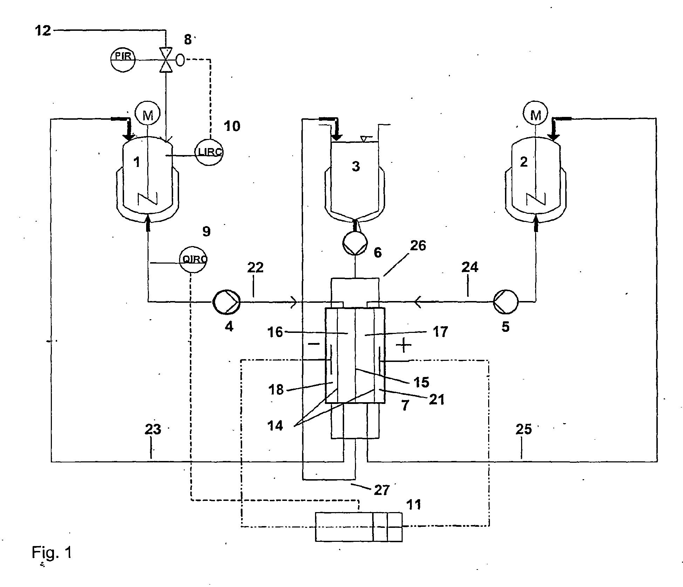

Die im nachfolgend beschriebenen Beispiel verwendete Anlage (Figur 1) besteht aus je einem temperierbaren Vorlagebehälter für Konzentrat 1, Permeat 2 und Elektrodenpuffer 3. Die Lösungen werden mittels Pumpen 4, 5, 6 über Vorlaufleitungen 22, 24, 26 und Rücklaufleitungen 23, 25, 27 rezirkuliert und durchströmen ein Elektrophoresetrennmodul 7. Die Drucküberlagerung im Gasraum des Konzentraums zur Einstellung des Permeatflusses über die Membran mit Stickstoff aus der Leitung 12 erfolgt mittels Nachdruckregler 8. Der Nachdruckregler wird wiederum über Füllstandssonde 10 geregelt.

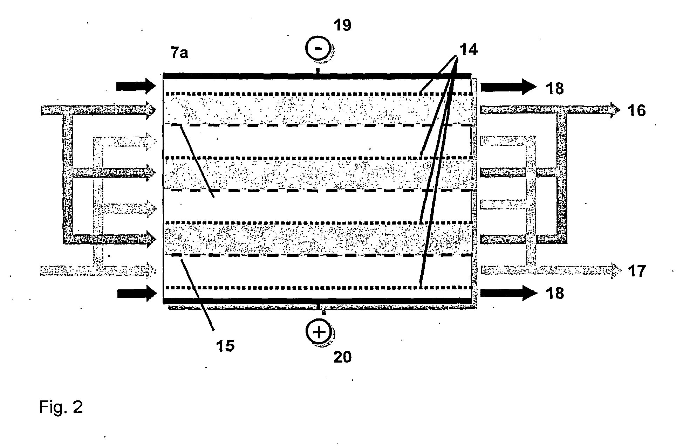

Die Elektrofiltrationsanlage enthält ein Modul 7a mit (vergleiche Fig. 2) mit jeweils vier parallelen Konzentraträumen 16a-d und Permeaträumen 17a-d. Die Konzentrat- 16 und Permeaträume 17 werden über Flüssigkeitsverteiler 28, 29 parallel angeströmt und durch Restriktionsmembranen 14 und Separationsmembranen 15 begrenzt. Konzentrat- und Permeaträume können hier nicht gezeigte Gitternetze oder Gewebe enthalten, die als Abstandhalter zwischen den Membranen und als Strömungsbrecher fungieren. Die Elektrodenräume 18, 21 werden parallel angeströmt und durch Restriktionsmembranen 14 begrenzt. Über Elektroden 19, 20 wird ein elektrisches Feld aufgebaut. das elektrische Feld kann wie in Figur 2 eingezeichnet oder auch entgegengesetzt aufgebaut werden. Die Regelung des elektrischen Feldes erfolgt über die gemessene Leitfähigkeit 9 in der Konzentratzuleitung 22 an der externen Automatisierungseinheit und wird an Netzteil 11 eingestellt. Die Anpassung des Permeatflusses über die Membran erfolgt durch Berechnung des aktüll anliegenden elektrischen Feldes im Upstream, ebenfalls über die gemessene Leitfähigkeit 9 in der Konzentratzuleitung 22 und über das Verhältnis aus zu jedem Zeitpunkt erreichter Ist- und gewünschter Sollfeldstärke und Übertragung dieses Verhältnisses auf die Permeatrate durch die externe Automatisierungseinheit.

**Beispiel:**

Die in Figur 1 gezeigte Anlage sowie das in Figur 2 skizzierte Modul 7a wurden zur Trennung und Aufkonzentrierung eines monoklonalen Antikörpers eingesetzt. Das produkthaltige Zellkulturmedium wurde nach der Fermentation mittels Ultrafiltration (Porengröße der Membran $2\mu m$) von Zellen und Zellteilen abgetrennt. Bei diesem Antikörper handelt es sich um einen IgG-Typ, der in einer Hybridomazelle exprimiert und in einem gerührten 0,8L-Bioreaktor fermentiert wurde. Der Antikörper hat einen isoelektrischen Punkt von 6,5 und eine Größe von 150kD.

Bei dem Modul 7a handelt es sich um eine Eigenentwicklung mit einer effektiven Membranfläche von $48cm^2$ pro Membranlage, die in Patentanmeldung DE A 0 40 07 848, Vorrichtung und Verfahren für die Membranelektrophorese und Elektrofiltration näher beschrieben ist.

Das Modul 7a umfasst dabei mindestens eine erste Halterplatte, einen ersten Elektrodenraum mit Elektrode, wenigstens je einen Eingangs- und Ausgangsraum, einen zweiten Elektrodenraum mit Elektrode und eine zweite Halterplatte, wobei die Räume gegeneinander durch flächige Zuschnitte von Membranen getrennt sind und wobei wenigstens die Membranen in ihren Randbereichen von einem Dichtrahmen in einem festgefügten Modul zusammengefasst sind, der Dichtrahmen Kanäle für die Zuführung und Abfiihrung von Flüssigkeiten besitzt mit davon abgehenden Durchbrechungen zu ausgewählten Räumen und wobei in mindestens einer Halterplatte Anschlusskanäle vorhanden sind, die mit den jeweiligen Kanälen im Dichtrahmen korrespondieren.

Es wurde ein TRIS/Zitronensäure-Puffer (34,4mM TRIS und 7,5mM Zitronensäure, pH8,0, Leitfähigkeit 1,53mS/cm) verwendet. Die Vorlage für Permeatlösung 2 wurde mit 1000mL und die der Elektrodenspüllösung 3 ebenfalls mit 1000mL Pufferlösung befiillt. Die Konzentrat-Vorlage wurde mit 1500mL Zellkulturüberstand (pH8) befiillt.

Der Versuch wurde bei Temperaturen von 6 bis 10°C durchgeführt.

Das Trennmodul ist mit Restriktionsmembranen 14 mit einer nominalen Trenngrenze von 10kDa sowie Separationsmembranen 15 mit einer nominalen Trenngrenze von 300kDa ausgerüstet.

Der Versuch wurde bei steigender Feldstärke bis zu einem maximalen Wert von 1500V/m durchgefiihrt, wobei an der konzentratseitigen Elektrode 20 ein negatives Potential angelegt wurde. Gleichzeitig wurde der Permeatfluss bis zu einem maximalen Wert von $30L/(h*m^2)$ gesteigert, wobei Feldstärke und Permeatfluss in einem konstanten Verhältnis standen. Die Volumenströme im Konzentrat- und Permeatkreislauf betrugen jeweils 320mL/min, entsprechend einer Überströmungsgeschwindigkeit von 5,2cm/s, der Volumenstrom im Elektrodenkreislauf 550mL/min. Die Antikörperkonzentrationen wurden mittels HPLC über eine Protein A Säule bestimmt. Die Gesamtproteinkonzentration als Maß für die Abreicherung der Nebenkomponenten wurde mittels BCA- (Bicinchinon- Säure) Methode und durch Erstellung von reduzierenden SDS-PAGE-Gelen analysiert.

Folgender Versuch wurde durchgeführt:

Elektrofiltration mit ansteigendem elektrischen Feld und im Verhältnis ansteigender Permeatrate über die Membran.

Tabelle 1 enthält die Versuchsparameter und Konzentrationsverläufe des Beispiels.

**Tabelle 1**

| Laufzeit | Feld-Stärke US E | Volumen | Nebenkomponenten Gesamtmasse | mAK GesamtMasse | Verhältnis mAK/NK | mAK Gesamt-Konzentration | Permeatrate P | Verhältnis P/E |
|---|---|---|---|---|---|---|---|---|
| min | V/m | mL | mg | mg | | mg/L | L/h*m$^2$ | (L/h*m$^2$)/(V/m) |
| 0 | 413 | 1458 | 1273 | 79 | 1,0 | 54 | | |
| 15 | 449 | 1453 | | | | | | |
| 30 | 458 | 1392 | 1150 | 78 | 1,1 | 55 | 12,8 | 0.028 |
| 45 | 548 | 1349 | | | | | 9,1 | 0,017 |
| 60 | 598 | 1292 | | 73 | | 56 | 12,0 | 0,020 |
| 75 | 644 | 1252 | | | | | 8,4 | 0,013 |
| 90 | 693 | 1192 | 894 | 69 | 1,2 | 57 | 12,6 | 0,018 |
| 105 | 782 | 1127 | | | | | 13,7 | 0,018 |
| 120 | 833 | 1054 | | 65 | | 60 | 15,4 | 0,018 |
| 135 | 972 | 971 | | | | | 17,5 | 0,018 |
| 150 | 1032 | 906 | 659 | 62 | 1,5 | 66 | 13,7 | 0,013 |
| 165 | 1210 | 810 | | | | | 20,2 | 0,017 |
| 180 | 1357 | 719 | | 59 | | 79 | 19,2 | 0,014 |
| 195 | 1340 | 550 | | | | | 35,6 | 0,027 |
| 210 | 1492 | 445 | 308 | 59 | 3,1 | 98 | 22,1 | 0,015 |
| 225 | 1491 | 350 | | | | | 20,0 | 0,013 |
| 240 | 1445 | 170 | 136 | 59 | 6,9 | 144 | 37,9 | 0,026 |

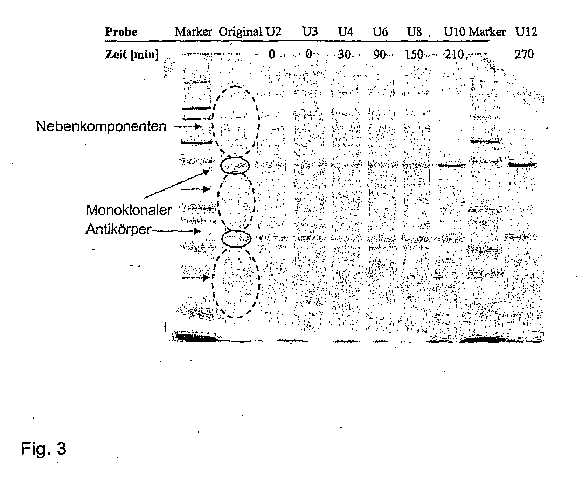

Fig 3 zeigt SDS-PAGE Gel unter reduzierenden Bedingungen. U2 bis U12 sind Upstreamproben zu verschiedenen Zeitpunkten des Versuchs.

Für die Abreicherung von Nebenkomponenten ergibt sich nach 240 Minuten ein massenbezogener abgereicherter Anteil von 0,89, bei einem Aufkonzentrierungsfaktor des Konzentrats von 8,6 und einer Ausbeute an Antikörper von 0,74.

Über den gesamten Verlauf von Beispiel 2 ergibt sich somit eine Selektivität von 7,3 mit

$m/m_0$ (HSA): massenbezogener Restanteil des HSA im Konzentrat     [-]

$m/m_0$ (IgG): massenbezogener Restanteil des IgG im Konzentrat     [-]

$$\psi = \frac{\ln[m/m_0\,(HSA)]}{\ln[m/m_0\,(IgG)]} : \qquad \text{Selektivität} \qquad\qquad [-]$$

## Patentansprüche

1. Verfahren zur Verbesserung der Selektivität und Produktivität bei der Aufreinigung von geladenen Makromolekülen und Partikeln aus Feedlösungen mit sich im Verfahren verändernden Leitfähigkeiten durch Elektrofiltration mittels Membranen im elektrischen Feld, **dadurch gekennzeichnet, dass** das Verhältnis aus oben genanntem elektrischen Feld und dem Permeatfluss durch die Membran konstant gehalten wird und dass das elektrische Feld auf der Feedseite der Membran (Feedkanal) in Abhängigkeit von der gemessenen Leitfähigkeit der Feedlösung geregelt wird.

2. Verfahren nach Anspruch 1 **dadurch gekennzeichnet, dass** das elektrische Feld 500 bis 5000 V/m beträgt.

3. Verfahren nach Anspruch 1 **dadurch gekennzeichnet, dass** der Permeatstrom zwischen Konzentrat- und Permeatkammer so eingestellt wird, dass ein Flüssigkeitsstrom durch die Separationsmembran gewährleistet wird.

4. Verfahren nach den vorhergehenden Ansprüchen, bei dem eine oder mehrere der folgenden Substanzen behandelt werden: Proteine, Peptide, DNA, RNA, Oligonucleotide, Oligo- und Polysaccharide, Viren, Virenbestandteile, Zellen, Zellenbestandteile, Enantiomere, Diastereomere.

5. Verfahren nach den vorhergehenden Ansprüchen, wobei es sich bei den Proteinen um monoklonale oder polyklonale Antikörper handelt.

6. Verfahren nach den vorhergehenden Ansprüchen, **dadurch gekennzeichnet, dass** die Membranen eine Porengröße von 1 bis 1000 nm aufweisen.

7. Verfahren nach Anspruch 6, **dadurch gekennzeichnet, dass** die Membranen eine Porengröße von 10 bis 500 nm aufweisen.

8. Verfahren nach den vorhergehenden Ansprüchen, **dadurch gekennzeichnet, dass** die Leitfähigkeit der Ausgangslösung zwischen 20 und 0,5 mS/cm, liegt.

9. Ein Verfahren nach einem der obigen Ansprüche, bei dem die verwendeten Elektrolyte eine Kombination aus schwachen Säuren und schwachen Basen, schwachen Säuren und starken Basen oder starken Säuren und schwachen Basen sind.

10. Ein Verfahren nach einem der obigen Ansprüche, bei dem die Elektrolytlösungen eine oder mehrere der folgenden Substanzen enthalten: Borsäure, Phosphorsäure, N-2-(Acetamido)-2-aminoethansulfonsäure, N-2-(Acetamido)iminodiessigsäure, Alanin, 2-Amino-2-methyl-1,3-propandiol, Ammoniak, N,N-Bis(2-hydroxyethyl)-2-aminoethansulfonsäure, N,N-Bis(2-hydroxyethyl)glycin, 2,2-Bis(hydroxyethyl)-iminotris(hydroxymethyl)methan, 2-(Cyclohexylamino)ethansulfonsäure, Essigsäure, Glycin, Glycylglycin, 2-[4-(2-Hydroxyethyl)1-piperazinyl]ethansulfonsäure, 3-[4-(2-Hydroxyethyl)1-piperazinyl]propansulfonsäure, Histidin, Imidazol, Milchsäure, 2-Morpholinoethansulfonsäure, 2-Morpholinopropansulfonsäure, Piperazin-1,4-bis(2-ethansulfonsäure), N-[Tris(hydroxymethyl)-methyl]-2-aminoethansulfonsäure, N-[Tris(hydroxymethyl)-methyl]glycin, Triethanolamin, Tris(hydroxymethyl)aminomethan, Zitronensäure.

**11.** Verfahren nach Anspruch 10, **dadurch gekennzeichnet, dass** die Elektrolytlösungen ein Puffersystem mit einem pH-Wert zwischen 3 und 11 bilden.

**12.** Verfahren nach einem der vorstehenden Ansprüche, **dadurch gekennzeichnet, dass** die Ausgangslösung ein Zellkulturüberstand einer Fermentation ist, der vorher einer Vorfiltration mittels einer Mikrofiltrationsmembran unterworfen wird.

**Claims**

**1.** Method for improving the selectivity and productivity in the purification of charged macromolecules and particles from feed solutions having conductivities which change in the course of the method by electrofiltration by means of membranes in an electric field, **characterized in that** the ratio of abovementioned electric field and the permeate flow through the membrane is kept constant and **in that** the electrical field on the feed side of the membrane (feed channel) is regulated as a function of the measured conductivity of the feed solution.

**2.** Method according to Claim 1, **characterized in that** the electric field is from 500 to 5000 V/m.

**3.** Method according to Claim 1, **characterized in that** the permeate flow between concentrate chamber and permeate chamber is adjusted so that a liquid flow through the separation membrane is ensured.

**4.** Method according to the preceding claims, in which one or more of the following substances are treated: proteins, peptides, DNA, RNA, oligonucleotides, oligo- and polysaccharides, viruses, virus constituents, cells, cell constituents, enantiomers, diastereomers.

**5.** Method according to the preceding claims, the proteins being monoclonal or polyclonal antibodies.

**6.** Method according to the preceding claims, **characterized in that** the membranes have a pore size of from 1 to 1000 nm.

**7.** Method according to Claim 6, **characterized in that** the membranes have a pore size of from 10 to 500 nm.

**8.** Method according to the preceding claims, **characterized in that** the conductivity of the starting solution is from 20 to 0.5 mS/cm.

**9.** A method according to any of the above claims, in which the electrolytes used are a combination of weak acids and weak bases, weak acids and strong bases or strong acids and weak bases.

**10.** A method according to any of the above claims, in which the electrolyte solutions contain one or more of the following substances: boric acid, phosphoric acid, N-2-(acetamido)-2-aminoethanesulfonic acid, N-2-(acetamido)iminodiacetic acid, alanine, 2-amino-2-methyl-1,3-propanediol, ammonia, N,N-bis(2-hydroxyethyl)-2-aminoethanesulfonic acid, N,N-bis(2-hydroxyethyl)glycine, 2,2-bis(hydroxyethyl)iminotris (hydroxymethyl)methane, 2-(cyclohexylamino) ethanesulfonic acid, acetic acid, glycine, glycylglycine, 2-[4-(2-hydroxyethyl)1-piperazinyl]ethanesulfonic acid, 3-[4-(2-hydroxyethyl) 1-piperazinyl]propanesulfonic acid, histidine, imidazole, lactic acid, 2-morpholinoethanesulfonic acid, 2-morpholinopropanesulfonic acid, piperazine-1,4-bis(2-ethanesulfonic acid), N-[tris(hydroxymethyl)methyl]-2-aminoethanesulfonic acid, N-[tris-(hydroxymethyl)methyl]glycine, triethanolamine, tris(hydroxymethyl)aminomethane, citric acid.

**11.** Method according to Claim 10, **characterized in that** the electrolyte solutions form a buffer system having a pH between 3 and 11.

**12.** Method according to any of the above claims, **characterized in that** the starting solution is a cell culture supernatant from a fermentation which has been subjected beforehand to a preliminary filtration by means of a microfiltration membrane.

**Revendications**

1. Procédé pour l'amélioration de la sélectivité et de la productivité dans la purification de particules et de macromolécules chargées à partir de solutions d'alimentation à conductivités variables dans le processus, par électrofiltration au moyen de membranes dans le champ électrique, **caractérisé en ce que** le rapport du champ électrique précité et du flux de perméat à travers la membrane est maintenu constant et **en ce que** le champ électrique sur la face alimentation de la membrane (canal d'alimentation) est réglé en fonction de la conductivité mesurée de la solution d'alimentation.

2. Procédé selon la revendication 1, **caractérisé en ce que** le champ électrique vaut de 500 à 5 000 V/m.

3. Procédé selon la revendication 1, **caractérisé en ce qu'**on ajuste le courant de perméat entre chambre de concentré et chambre de perméat de manière à assurer un courant de liquide à travers la membrane de séparation.

4. Procédé selon l'une quelconque des revendications précédentes, dans lequel on traite une ou plusieurs des substances suivantes : protéines, peptides, ADN, ARN, oligonucléotides, oligo- et polysaccharides, virus, constituants viraux, cellules, constituants cellulaires, énantiomères, diastéréo-isomères.

5. Procédé selon l'une quelconque des revendications précédentes, dans lequel les protéines consistent en des anticorps monoclonaux ou polyclonaux.

6. Procédé selon l'une quelconque des revendications précédentes, **caractérisé en ce que** les membranes ont une taille de pore de 1 à 1 000 nm.

7. Procédé selon la revendication 6, **caractérisé en ce que** les membranes ont une taille de pore de 10 à 500 nm.

8. Procédé selon l'une quelconque des revendications précédentes, **caractérisé en ce que** la conductivité de la solution de départ est comprise entre 20 et 0,5 mS/cm.

9. Procédé selon l'une quelconque des revendications ci-dessus, dans lequel les électrolytes utilisés consistent en une association d'acides faibles et de bases faibles, d'acides faibles et de bases fortes ou d'acides forts et de bases faibles.

10. Procédé selon l'une quelconque des revendications précédentes, dans lequel les solutions électrolytiques contiennent une ou plusieurs des substances suivantes : acide borique, acide phosphorique, acide N-2-(acétamido)-2-aminoéthane-sulfonique, acide N-2-(acétamido)iminodiacétique, alanine, 2-amino-2-méthyl-1,3-propanediol, ammoniac, acide N,N-bis(2-hydroxyéthyl)-2-aminoéthanesulfonique, N,N-bis(2-hydroxyéthyl)glycine, 2,2-bis(hydroxyéthyl)-iminotris(hydroxyméthyl)méthane, acide 2-(cyclohexylamino)éthanesulfonique, acide acétique, glycine, glycylglycine, acide 2-[4-(2-hydroxyéthyl)1-pipérazinyl]éthanesulfonique, acide 3-[4-(2-hydroxyéthyl)1-pipérazinyl] propanesulfonique, histidine, imidazole, acide lactique, acide 2-morpholinoéthanesulfonique, acide 2-morpholinopropanesulfonique, acide pipérazine-1,4-bis(2-éthane-sulfonique), acide N-[tris(hydroxyméthyl)-méthyl]-2-aminoéthanesulfonique, N-[tris(hydroxyméthyl)-méthyl]-glycine, triéthanolamine, tris(hydroxyméthyl)amino-méthane, acide citrique.

11. Procédé selon la revendication 10, **caractérisé en ce que** les solutions électrolytiques forment un système tampon à un pH compris entre 3 et 11.

12. Procédé selon l'une quelconque des revendications précédentes, **caractérisé en ce que** la solution de départ est un surnageant de culture cellulaire d'une fermentation, qui est soumis au préalable à une préfiltration au moyen d'une membrane de microfiltration.

Fig. 1

Fig. 2

| Probe | Marker | Original U2 | U3 | U4 | U6 | U8 | U10 | Marker | U12 |
|---|---|---|---|---|---|---|---|---|---|
| Zeit [min] | | 0 | 0 | 30 | 90 | 150 | 210 | | 270 |

Nebenkomponenten →

Monoklonaler
Antikörper →

Fig. 3

## IN DER BESCHREIBUNG AUFGEFÜHRTE DOKUMENTE

**In der Beschreibung aufgeführte Patentdokumente**

- US 3079318 A
- US 3989613 A, Gritzner
- US 5087338 A
- EP 0091784 A2
- US 5437774 A

- WO 9962937 A
- AU 601040 A
- DE 10253483 A
- DE 04007848 A
- US 5429746 A, Shadle

**In der Beschreibung aufgeführte Nicht-Patentliteratur**

- Electrically enhanced crossflow membrane filtration of oily waste water using the membrane as a cathode. **Huotari H.M. et al.** Journal of Membrane Science. Elseviers Science Amsterdam, 24. April 1999, vol. 156, 49-60